# EUROPEAN PATENT SPECIFICATION

(11) **EP 4 259 290 B1**
(45) Date of publication and mention of the grant of the patent: **30.10.2024**
(21) Application number: 21839349.4
(22) Date of filing: 09.12.2021
(51) Int. Cl.: A62B 23/02, A41D 13/11, A62B 18/02, A62B 18/08, B29C 45/14, B29C 45/17, B29K 21/00, B29K 105/04, B29L 31/14, B29L 31/26, B29L 12/00, B29L 22/00

(54) **METHOD OF MANUFACTURING A FILTER MASK AND CORRESPONDING FILTER MASK**
HERSTELLUNGSVERFAHREN VON EINER FILTERMASKE UND ENTSPRECHENDE FILTERMASKE
MÉTHODE DE FABRICATION DE MASQUE À FILTER ET MASQUE CORRESPONDANT

(30) Priority: 09.12.2020 GB 202019406; 16.07.2021 GB 202110291
(43) Date of publication of application: 18.10.2023
(60) Divisional application: 24201567.5
(73) Proprietor: Intersurgical AG, 9490 Vaduz (LI)
(72) Inventor: MILLER, Andrew Neil, Wokingham Berkshire, RG41 2RZ (GB); LEARY, Matthew James William, Wokingham Berkshire, RG41 2RZ (GB); PAYNE, Simon Robert, Wokingham Berkshire, RG41 2RZ (GB); BOTTOM, David Simon, Wokingham Berkshire, RG41 2RZ (GB)
(74) Representative: Adamson Jones
(86) International application number: PCT/EP2021/085080
(87) International publication number: WO 2022/122974

(56) References cited:
- EP-A1- 1 582 231
- CN-A- 111 109 715
- DE-U1- 202013 011 420
- US-A1- 2004 074 498
- US-A1- 2012 125 341

## Description

The present invention relates to a method of manufacturing a filter mask and a corresponding filter mask.

Face masks that filter out particulate substances from the air, ie filter masks, are typically one of two types. In particular, the face mask may have a mask body that itself functions to filter out particulate substances from the air, and this type of filter mask is typically lightweight and relatively inexpensive, so as to be single-use and disposable. Alternatively, the face mask may have a mask body adapted to receive a replaceable filter cartridge, so as to be reusable.

Filter masks may be worn in work or public environments to protect from dust, smoke and other noxious substances, and to protect from pathogens, such as viruses or bacteria. In a healthcare environment, for example, filter masks are used to prevent the inhalation of pathogens from the environment by preventing the passage of particulates, eg droplets and aerosols, through the mask. Filter masks may also protect others from exhalation of pathogens by the wearer.

Filter masks typically comprise a filter, such as a sheet of textile or nonwoven filter material, and a means for retaining the mask on the wearer's head, for example by loops of elastic which are placed around the ears. In use, the filter covers the user's mouth and nose, or air is otherwise directed through the filter, so that any air inhaled by the wearer passes through the filter. In order to protect others from exhalation of pathogens by the wearer, the filter may be arranged so that any air exhaled by the wearer passes through the filter.

A conventional method of manufacturing a filter mask and a corresponding filter mask are disclosed in US 2012/125341 A1.

Filter masks worn in work environments, such as by healthcare professionals, must stay securely fitted to the face and be comfortable to wear for a substantial period of time in an environment in which the risk of contamination may be high. Such masks must closely fit the wearer's face, and preferably form a seal against the face so that inhaled and exhaled air cannot pass around the edge of the mask.

Filter masks are commonly single-use products, produced and sold in high volume. For products of this type to be commercially viable, the cost and complexity of manufacture must be kept to a minimum.

There has now been developed a face mask for filtering air and a method for its production which overcomes or substantially mitigates the above mentioned and/or other problems associated with the prior art.

According to the invention there is provided a method of manufacturing a filter mask according to claim 1.

The injection moulding of the sealing member in the method according to the invention may be overmoulding. By "overmoulding" is meant, in the context of the invention, an injection moulding process in which a first material is moulded onto a second material to form a single item, and where the first material may partially or wholly cover the second material.

The method of manufacturing a filter mask according to the invention is advantageous principally because the step of injection moulding of the sealing member fixes the sealing member to the support frame and the sheet of filtering material, which fixes the relative positions of these components without requiring any further assembly or moulding steps. Face masks, in particular filter masks, are products which are produced at high volume and sold at low cost. As the profit margin on each individual item is low, reducing the cost and complexity of the manufacturing method is key to producing a viable product.

According to a further aspect of the invention, there is provided a filter mask manufactured according to the method described above. According to a further aspect of the invention, there is provided a filter mask according to claim 12.

The support frame and the filter may together define a mask body, which forms a cavity for the accommodation of the wearer's mouth and nose. The mask body may be curved in at least the transverse plane.

The support frame may be made in a preceding step of the method of the invention. The support frame may be made in a preceding step by injection moulding. Thus, the method may further comprise a preceding step in which the support frame is formed by injection moulding. The method may further comprise a step of transferring the support frame from a first mould in which the support frame is injection moulded to a second mould in which the sealing member is injection moulded.

The support frame may be made of a plastics material, such as polypropylene, poly(styrene-butadiene-styrene) (SBS), polycarbonate, plastomers, thermoplastics, thermoplastic elastomers, thermosets or blends or combinations of the foregoing.

The support frame may have an external face and an internal face, the internal face, in use, facing the wearer and the external face, in use, facing away from the wearer.

The support frame may support at least a portion of the filter, eg by preventing distortion of the sheet of filtration material. The support frame may provide rigidity to the mask.

The support frame may take the form of a rim, for example that matches the shape of the filtering material. The rim may have the form of a closed loop. The rim may define an aperture through which inhaled and/or exhaled gases flow, in use. When the filter is placed onto the support frame, the support frame may extend about the periphery of the filter. The support frame may comprise a shoulder extending from an inner edge of the rim, providing a surface on which the filter can be located, facilitating retention of the filter in the mould prior to injection moulding of the sealing member. The filter may be located on the internal face of the support frame.

Alternatively, or additionally to the rim, the support frame may comprise one or more ribs, eg a plurality of ribs, which extend across a surface of the filter, eg for providing support to the filter and rigidity to the mask.

The external face of the support frame may further comprise a shield which, in a filter mask according to the invention, extends over at least part of a surface of the filter. The shield may be separated from the filter, such that air may flow between the shield and the filter, eg before the air is drawn through the filter by the wearer during inhalation. There may therefore be a space or void between the shield and the filter. An inlet aperture, through which air may be drawn by a wearer inhaling, may be defined between an edge of the shield and a surface of the filter.

The shield may provide a barrier upon which particles in the environment impinge. The shield may therefore reduce the quantity of particles incident on the filter during use, and hence may extend the effective life of the filter and hence of the filter mask. The separation between the shield and the filter enables incoming air to flow through the filter across its entire surface, even where the shield is impermeable or substantially impermeable. The shield may additionally provide reinforcement to the mask, preventing longitudinal collapse of the mask, and keeping it in the correct place relative to the wearer's nose and chin.

The shield may be formed of plastics material. This may form a solid barrier to deflect particulates, and reduce the risk of physical damage to the filter by providing a protective barrier. The shield may form a rigid barrier.

The shield may alternatively be a composite shield formed of two or more components. The composite shield may comprise a sheet of filter material (the shield filter) and a shield frame, the shield filter overlaying and being affixed to the shield frame. The shield frame may be a plastic frame, and may form part of the support frame. The shield frame may comprise a shield rim substantially the same shape as the intended shield and/or the shield filter, the shield rim being affixed to the periphery of the shield filter. Such a composite shield actively absorbs and blocks contaminants, and may reduce the build-up of humidity in the mask, increasing comfort for the wearer. The composite shield may be removably fixed to the mask, such that it can be replaced if it becomes saturated; either the entire composite shield may be removeable and replaceable, or just the sheet of filter material may be replaceable.

According to an example, there is provided a filter mask comprising a support frame, a filter and a sealing member, the support frame retaining the filter, such that ambient air is drawn through the filter on inhalation by a wearer, wherein the support frame includes a shield that extends over at least part of an outwardly-facing surface of the filter, wherein the shield is separated from the filter, such that air flows, in use, between the shield and the filter before the air is drawn through the filter by the wearer during inhalation.

According to an example, there is provided a filter mask comprising a support frame, a main filter and a sealing member, the support frame retaining the main filter, such that ambient air is drawn through the main filter on inhalation by a wearer, wherein the support frame includes a shield frame, the shield frame carrying a shield filter which extends over at least part of an outwardly-facing surface of the main filter, wherein the shield frame is separated from the main filter, such that air flows, in use, between a peripheral edge of the shield frame and the main filter.

In the following paragraphs, unless indicated otherwise references to the "shield" refer both to a shield formed of a plastics material, and to a composite shield formed of a shield frame and shield filter. In addition, unless indicated otherwise references to the "filter" refer to the main filter, that is, the filter retained by the support frame, and references to the "shield filter" refer to a filter forming part of a composite shield.

The support frame may comprise a rim and a shield, or a rim and a shield frame, and the shield or shield frame may be offset from the rim, eg in the transverse plane. The rim may extend about and/or over the periphery of the filter, and the shield or shield frame may be offset from both the rim and the filter. The rim and shield or shield frame may be connected together by one or more connection members, which may extend between the shield and the rim, and fix their relative positions. For example, connection members may extend between the rim and the shield or shield frame at three, or four, or five points, and these points may be regularly spaced about the rim.

The shield may be offset from the filter, eg in the transverse plane, and may have a shape corresponding to the shape of the filter, such that the surfaces of the filter and the shield are parallel.

There may be a spacing having an average size of 0.1 - 5cm, or of 0.2 - 2.5cm, or of 0.2 - 1.5cm, or of 0.3 - 1cm between the shield and the filter, measured along a line normal to the surface of the shield. This spacing between the shield and the filter may be maintained through one or more ribs extending across at least part of the surface of the shield that faces the filter. The ribs may be straight or arcuate. The ribs may contact the filter, preventing the shield from coming into contact with the filtering material and thus maintaining a flow of air between the shield and the filtering material, even during movement by the wearer.

The surface area of the shield may be at least 50%, at least 60%, at least 70%, or at least 80%, or at least 90%, or at least 95% of the surface area of the filter. The surface area of the shield may be up to 110% of the surface area of the filter, or up to 105%, or up to 100%, or up to 95% of the surface area of the filter. The shield may extend over at least 50%, at least 60%, at least 70%, or at least 80%, or at least 90%, or at least 95%, or up to 100% of the surface area of the filter.

The shield may extend over the entire surface area of the filter, or may extend beyond the periphery of the filter such that the shield overhangs the edges of the filter. Alternatively, the shield may extend over less than 100% of the surface area of the filter, such that a portion of the filter is exposed at the periphery of the shield. Alternatively, or additionally, the shield may have apertures formed therein through which the sheet of filtering material is exposed, which may assist the flow of air and help to evenly distribute the particle load on the filter. The shield may alternatively have no apertures, but form a continuous barrier. Thus, the shield may comprise a continuous surface that extends across a majority of the surface of the filtering material.

There may be a space between an edge of the shield and a rim of the support frame through which the filter is exposed to the environment, caused by the horizontal offset of the shield from the filter and/or the shield extending over less than 100% of the surface area of the filter. The offset of the shield from the filter and/or the partial covering of the filter by the shield permit the flow of air around the shield and through the filter, ensuring that the whole surface area of the filtering material is used while the wearer is breathing.

At the same time, the shield itself protects a portion, or a majority, of the filter. The shield may catch a proportion of airborne particulates, eg aerosol particles and/or other contaminants, which contact the mask, preventing them from contacting with filter. This prolongs the life of the mask by lengthening the time it takes for the filter to become saturated. The shield also reduces the risk of damage to the more delicate filter, and prevents or deters the wearer from touching the potentially contaminated filter whilst handling the mask.

At least part of the periphery of the shield may be separated from the rim of the support frame, thereby defining one or more inlet apertures through which air is drawn on inhalation by the wearer. The one or more inlet apertures may be arranged in a loop or partial loop, so as to enable occlusion by the wearer's fingers for performing a fit test. In particular, prior art tests for the fit of a face mask on a wearer typically involve expensive test apparatus or are not easy for a wearer to perform themselves. The arrangement of the one or more inlet apertures in a loop or partial loop, so as to enable occlusion by the wearer's fingers, enables a wearer to then test whether any air leaks into the mask when the one or more inlet apertures are occluded, thereby testing the fit of the mask. In particular, a wearer should not be able to inhale when the one or more apertures are occluded if the face mask fits effectively.

According to an example, there is provided a filter mask comprising a support frame, a filter and a sealing member, the support frame retaining the filter, such that ambient air is drawn through the filter on inhalation by a wearer, wherein the filter mask includes a shield that extends over at least part of an outwardly-facing surface of the filter, and at least part of the periphery of the shield being separated from the support frame, and one or more inlet apertures being defined between the shield and the support frame, through which air is drawn on inhalation by the wearer.

The separation between the periphery of the shield and the support frame is preferably less than the width of the average person's finger, eg index finger, for either an adult for an adult mask or a child for a child's mask. For example, the separation between the periphery of the shield and the support frame may be less than 2cm, or less than 1.5cm or less than 1cm.

The one or more inlet apertures may be arranged around the entire periphery of the shield, but may be separated by members connecting the support frame and the shield. The one or more inlet apertures in the nasal region and/or the chin region may extend along curved paths from an angled apex in a central region of the mask, outwardly towards the sides of the mask, so as to receive the fingers of the wearer during a fit test, the fingers meeting at the angled apex. Alternatively, or in combination, the one or more inlet apertures in the nasal region and/or the chin region may extend a regular curved path from one side of the mask to the other, so as to receive the fingers of the wearer during a fit test, the fingers meeting at a midpoint of the curved path. The one or more inlet apertures in the side regions of the mask may be aligned with the longitudinal axis of the mask and the wearer, ie the axis from the nose region to the chin region, and may be generally linear in form, so as to receive the part of the hand between the index finger and thumb of the wearer during a fit test, for example.

The one or more inlet apertures may alternatively be arranged around a portion of the periphery of the shield. In particular, inlet apertures may be arranged around the cheek regions and/or the chin region, with no inlet aperture present in the nasal region. This arrangement may direct exhaled airflow away from the wearer's eyes, increasing comfort for the wearer and reducing or preventing a wearer's glasses from becoming misted. With the apertures in this arrangement, the fit test described in the preceding paragraph may still be performed.

Alternatively, the mask may be provided with a cover for the one or more inlet apertures, which enables the apertures to be occluded without the wearer using their fingers for that purpose. Thus, there is also provided a kit of parts comprising the filter mask according to the aspect of the invention described above and a cover for the one or more inlet apertures, which enables the apertures to be occluded to perform a fit test.

Further arrangements for facilitating a fit test include means for generating a smell on the mask, which would be filtered out by the filter. For example, the mask may include an element for emitting a smell on an exterior side of the filter and a cover for inhibiting the smell before use, the cover being at least partially removed, eg peeled, scratched, etc, to at least partially expose the element for emitting a smell. A wearer should not be able to detect the smell if the face mask fits effectively.

According to an example, there is provided a filter mask comprising a support frame, a filter and a sealing member, the support frame retaining the filter, such that ambient air is drawn through the filter on inhalation by a wearer from an exterior side of the filter to an interior side of the filter, wherein the filter mask includes an element for emitting a smell on the exterior side of the filter.

The filter mask may comprise a cover for inhibiting the smell before use, the cover being at least partially removable to at least partially expose the element for emitting a smell. The cover may reduce the risk of the smell being transferred to other parks of the filter mask, eg on the interior side of the filter. Furthermore, the cover can be removed once the mask has been fitted to ensure that the fit test is not affected by the wearer detecting the smell before the test.

The filter may be sufficiently impermeable to the smell that the wearer does not detect the smell in air drawn through the filter. The element may be exposed for emitting a smell on the exterior side of the filter only, such that the wearer should not be able to detect the smell if the face mask fits effectively.

The element for emitting a smell may comprise or be located on a portion of the support frame, for example on the shield or on a portion of the shield, or the element for emitting the smell may be applied to the entire support frame. If the element were applied to the entire support frame, any portions of the support frame on an interior side of the filter may need to be covered to inhibit the smell, eg by overmoulding a cover of thermoplastic elastomer.

The support frame may include a shield that extends over at least part of an outwardly-facing surface of the filter, and at least part of the periphery of the shield being separated from the support frame. One or more inlet apertures may be defined between the shield and the support frame, through which air is drawn on inhalation by the wearer,

The support frame is typically formed of a plastics material that retains its shape in use. The support frame may comprise a rim and/or a shield and/or one or more connection members. Where multiple components are present the entire support frame may be integrally formed, eg by injection moulding, or the support frame may be formed in two or more pieces which are connected together eg by gluing, welding or mechanical fastening. Thus, where the support frame comprises a rim and a shield, the rim and shield may be integrally formed in one injection moulding step, or the rim and the shield may be formed separately before being bonded together either chemically or mechanically.

The filter may be formed from a filter substrate that is sufficiently dense to prevent the passage of a majority of airborne particles, such as dust particles, droplets and aerosols. The mask of the present invention may filter out at least 80%, or at least 85%, or at least 90%, or at least 95% or at least 99% of airborne particles.

In countries including the UK and those in the European Union, the FFP standards specify requirements for filtering masks as respiratory protective devices. The EN 149 standard defines three classes of filter efficiency, namely FFP1, FFP2 and FFP3. The mask of the present invention may be a FFP3 mask, for example, which under the EN 149 standard filters out at least 99% of airborne particles at 95 L/minute airflow, and with a total inward leakage of less than 2%. The mask of the present invention may be a FFP2 mask, which filters out at least 94% of airborne particles at 95 L/minute, and with a total inward leakage of less than 8%. In the USA, the U.S. National Institute for Occupational Safety and Health (NIOSH) provides an N95 classification of air filtration, and the mask of the present invention may be an N95 mask, meaning that it filters at least 95% of airborne particles.

Materials suitable for use as the filter substrate are known in the art, and any suitable material may be used for the sheet of filtering material in the present invention. For example, the filter may be a nonwoven, electrostatic, meltblown, spunbond, textile, nanofiber or nanoweb material, or be formed of a glass fibre media. The filter may comprise a plurality of layers to enhance its performance and increase comfort for the wearer. Thus, the filter may comprise one or more layers selected from nonwoven, electrostatic, meltblown, spunbond, textile, nanoweb and/or nanofiber material. For example, the filter may comprise layers of nonwoven, electrostatic and meltblown material, together with a textile layer to increase comfort for the wearer and provide structure to the filter. The filter may be in any appropriate configuration, for example the surface of the filter may be smooth, eg it may be flat or planar, or the filter may be pleated. The filter (pleated or non-pleated) may have a flat or planar region about its periphery to facilitate the overmoulding process. The filter may further comprise one or more additives to enhance its effectiveness in reducing or preventing the transmission of pathogens, such as antimicrobial agents.

The use of a pleated filter increases the surface area of the filter, improving the effectiveness of the filter. Pleating the filter may increase the surface area by 5-50%, or by 5-30%, or by 10-20% compared to an unpleated filter. To accommodate a pleated filter, the shape of the support frame may correspond to the shape of the pleated filter, ensuring a close fit between the two components and preventing leaks. For example, where the filter abuts a shoulder or rim on the support frame, the shoulder or rim may comprise a series of corrugations which correspond to the pleats in the filter such that, when the filter contacts the shoulder or rim, the corrugations nest with the pleats of the filter to ensure a close fit. The filter may be pleated prior to it being located in the mould with the support frame, and overmoulding of the sealing member to fix the sealing member, support frame and filter together. Alternatively, compression of an unpleated (ie a flat or planar) filter between the corrugated rim or shoulder of the support frame and a correspondingly shaped mould may create the pleats in the filter, prior to overmoulding of the sealing member to fix the sealing member, support frame and filter together.

The filter that is located in the mould in the method of the present invention may comprise filter substrate only, and may be flexible in form. The filter may include no support for the filter substrate until fixed to the support frame and the sealing member.

The filter may be cut to the correct shape from a larger sheet of filter substrate, for example using a die. Where the filter is formed from a plurality of layers, the filter may be cut to size and the edges sealed in a single hot stamp process, in which heat is used to seal the peripheral edges of the seal during the cutting process. The sealed periphery, in use, prevents ingress of moisture or contaminants to the interior of the filter material, between the layers. The size and shape of the filter may correspond to the size and shape of at least a portion of the support frame. For example, the size and shape of the filter may correspond to the size and shape of the rim of the support frame, such that the filter can be positioned on the rim, eg on a shoulder inwardly extending from the rim, such that the support frame extends about the periphery of the filter.

The filter may be brought into contact with the support frame before the support frame and filter are together located in the mould. For example, the filter may be located on the rim of the support frame before the support frame and filter are together located in the mould. Alternatively, the support frame may be placed into the mould before the filter is positioned on the support frame. In either case, both the support frame and the filter are located in the mould prior to injection moulding of the seal member. Preferably, the support frame and the sheet of filtering material are not fixed or bonded together prior to locating in the mould.

The seal member is adapted to fit snugly against the wearer's face, and is overmoulded about both the filter and the support frame. Thus, the filter and the support frame are located in the mould, and their relative positions are fixed by injection moulding of the seal member. This fixes the support frame, filter and sealing member together in a single step, without the need for additional adhesive or overmoulding steps, thereby reducing time and costs.

To fix the filter and support frame together in a single overmoulding step, the filter may be positioned on the external or internal face of the support frame, eg on the rim, such that the edges of the sheet of filtering material and support structure are substantially aligned. During injection of the polymeric material that forms the sealing member, a portion of the polymeric material may envelop the aligned edges of the sheet of filtering material and the support structure. Thus, the peripheral edges of both the filter and the support frame, or rim of the support frame, may be enclosed within a portion of the polymeric material that forms the sealing member.

Alternatively, during overmoulding of the seal the injected material may be directed between the filter and the support frame, such that the injected material forms an adhesive layer between the filter and support frame about the periphery of the filter. The periphery of the filter may be enveloped by the injected material, which then bonds to the support frame (eg to the rim of the support frame). The injected material may be absorbed into the filter about its periphery, increasing the bond between the seal and the filter and providing an additional protection against ingress of moisture between layers of the filter material.

By "substantially aligned" is meant that the edges of the filtering material and support structure are located close enough together that they can be enveloped or otherwise adhered together by a portion of the sealing member.

The sealing member may be formed of an elastomeric material, and suitable materials for use in making sealing members for face masks are known in the art. For example, the sealing member may be formed of a thermoplastic elastomer (TPE) or a thermoset elastomer, such as liquid silicone rubber.

The sealing member may be shaped to fit snugly and to seal against the wearer's face, such that all of the air inhaled by the wearer passes through the filtering material. To increase comfort and sealing effectiveness, the sealing member may be in the form of a cushion. That is, the sealing member may form a resiliently compressible region between the support frame and the wearer's face, increasing comfort and improving the seal.

As the effectiveness of the mask increases with improvement in fit (and reduction in leaks), it is useful to have a mask that may accommodate faces of different sizes, and which remains effective even where the wearer is moving and carrying out an active role, such as in a filter mask for use by a healthcare professional.

Any aspect of the support frame and/or seal may comprise one or more additives to help increase the effectiveness of the mask in reducing or preventing the spread of bacteria or other pathogens. In particular, an additive containing silver ions (for example in the form of Silver Knight^{™}) may be incorporated into the plastic of the support frame and/or seal. Silver ions have been found to catalyse the deactivation of pathogenic bacteria and prevent their proliferation, reducing microbial growth within and on the surface of the mask.

According to an example, there is provided a filter mask comprising a support frame and a sheet of filtering material, the support frame and the sheet of filtering material defining a mask body adapted to provide a cavity in use about the mouth and nose of a wearer, the filter mask includes a sealing member depending from at least a portion of a peripheral edge of the mask body, the sealing member comprising both an inwardly and outwardly depending lip portion relative to the peripheral edge of the mask body.

The sealing member may extend around the entire peripheral edge of the mask body. The portion of the peripheral edge of the support frame about which the inwardly and outwardly depending lip portions extend may comprise a chin portion. The inwardly depending lip portion may extend around a majority of the peripheral edge of the support frame. The outwardly depending lip portion may extend around only a part of the peripheral edge of the support frame, eg in the chin region.

The inwardly depending lip portion may be generally planar in form. The inwardly depending lip portion may take the form of an upstanding wall which follows the contour of the peripheral edge of the body. The inwardly depending lip portion may include discontinuities therein that facilitate deformation of the inwardly depending lip portion.

The outwardly depending lip portion may have a contact surface that is concave in shape. The outwardly depending lip portion may be shaped so as to provide a sill or shelf formation beneath the wearer's mandible. A proximal portion of the outwardly depending lip portion may engage the chin of the wearer, while a distal portion curves and extends under the chin of the wearer. The outwardly depending lip formation may be shaped in the form of a chin cup. The chin cup may, in use, accommodate at least a portion of the chin of the wearer. The outwardly depending lip portion may be shaped so as to provide both a front lip portion and also an underside lip portion.

The inwardly depending lip portion may contact a lower portion of the wearer's face, eg the wearer's chin, to form a seal. Where a user has a larger face height, the outwardly depending lip portion may pass closely beneath the mandible of a wearer, providing an additional seal. For all wearers, the outwardly depending lip portion may provide an alignment guide for placing the mask on the face, and help to keep the mask in the correct position on the face despite movement of the jaw during use.

The periphery of the support frame in the vicinity of the inner and outer lip portions may be shaped from a central point or region of the mask by a first radius. The inwardly depending lip portion typically has an inner edge of radius which is smaller than the first radius. The outwardly depending lip portion typically has an outer edge of radius which is larger than the first radius.

The nasal portion of the sealing member may comprise an inwardly depending lip portion, which may present a convex surface for contact with a wearer's face. The nasal portion of the sealing member may include reinforcement formations, eg portions of increased thickness, on each side of the nose, and may have a reduced thickness, for example, in the nasal bridge region.

The sealing member and, therefore, the filter mask, may also extend laterally across the wearer's cheeks, such that a seal is formed between the mask seal and the tissue of the cheeks, and the internal cavity of the mask extends over the cheeks.

During speech, the majority of motion in the face is in the jaw, including vertical separation of the nose and chin, while there is a relatively small amount of movement in the cheeks. In addition, the tissue of the cheeks is softer and more compliant than the tissue of either the nose or chin. This means that a lower degree of flexibility is required in the sealing member to achieve an effective seal with the cheek tissue than with other parts of the face.

According to an example, there is provided a face mask comprising a mask body that defines a cavity for accommodating the nose and mouth of a wearer and from which a wearer inhales respiratory gases, in use, and a sealing member depending from the mask body for engagement with the wearer's face, the mask body comprising a nasal portion for accommodating the nose of the wearer, a mouth portion for accommodating the mouth of the wearer, and cheek portions extending laterally across the wearer's cheeks.

The cheek portions may extend laterally across the wearer's cheeks between the upper edges of the malar bones and the lower edge of the mandible of the wearer. The cheek portions may each have a first edge that extends from a left- or right-side edge of the nasal portion, such that the first edge of the cheek portion is accommodated over the malar bone of the wearer. The first edge may extend along an arcuate path. The first edge may be a concave exterior edge. The transition between the first edge of the cheek portion and the left- or right-side edge of the nasal portion may be arcuate in form.

The cheek portions may each have a second edge that extends from a left- or right-side edge of the mouth portion, such that the second edge of the cheek portion is accommodated above the lower edge of the mandible of the wearer. The second edge may extend along an arcuate path. The second edge may be a concave exterior edge. The transition between the second edge of the cheek portion and the left- or right-side edge of the mouth portion may be arcuate in form.

The angle between the first and second edges of each cheek portion may increase with increasing lateral position, and the first and second edges may become substantially parallel at a distal end of the cheek portion, eg proximate to the wearer's ear. An end edge of each of the cheek portions may be generally linear. A distal region of each cheek portion may be generally rectangular in shape. This may increase the surface area of the mask body that is covering the wearer's cheeks.

The mask body may extend a greater distance in a transverse direction across the face of the wearer, relative to the distance the mask body extends in the longitudinal direction across the face of the wearer. The greater distance may be relative to the surface of the face of the wearer, ie the distances may be dimensions along the surface of the face, as opposed to linear dimensions.

The mask body may extend in a greater distance in a transverse direction, relative to the distance the mask body extends in a longitudinal direction. The greater distance may be relative to the surface of the mask, ie the distances may be dimensions along or parallel to the surface of the mask, as opposed to linear dimensions.

The portions of the mask body which accommodate the wearer's nose and mouth may have an exterior surface with a generally arcuate shape, eg in the transverse plane, while the portion which accommodates the wearer's cheeks may have an exterior surface that is substantially more planar in shape, eg in the transverse plane. The filter mask, and the internal cavity of the filter mask, may extend laterally across the wearer's cheeks, such that an edge of the mask is situated, in use, within 5cm, or within 4 cm, or within 3cm, or within 2cm of the wearer's ears. Thus, the mask may substantially cover the wearer's cheeks.

Where the face mask is a filter mask, the mask body may be defined by the support frame and the filter. The sealing member may extend about the periphery of the support frame, eg about the periphery of the rim. The shape of at least a portion of the support frame and of the filter may correspond to the shape of the proximal edge of the sealing member. The support frame and the filter may extend laterally across the wearer's cheeks.

The mask body of the face mask may extend laterally across the wearer's cheeks. The sealing member of the face mask may extend laterally across the wearer's cheeks. The portions of the mask body which extends across the wearer's cheeks may form a shallower cavity between the mask and the wearer's face than the portion of the mask which extends over the wearer's nose and mouth.

The extension of the mask over the cheeks increases the surface area of the sheet of filtering material, thus increasing the volume of air which can pass through the filter. At the same time, the shallower cavity in this region of the mask reduces the dead space within the mask.

The filter mask according to the invention may include means for securing the mask to the wearer, in use. Such means may include one or more cords or straps, for example an elasticated cord or strap, that is fitted around the wearer's head to urge the filter mask against the face of the wearer.

The cord or strap may be formed as a separate component or components, and subsequently be attached to the filter mask either by insertion through a hole or slot and knotting or sealing, or by bonding the cord or strap to the mask. The cord or strap may be bonded to the mask by an adhesive or chemical bond, or by overmoulding. The cord(s) or strap(s) may be worn over the ears of the wearer, or around the back of the wearer's head. This may help prevent a mask from slipping down and/or rising up a wearer's face in use.

Two cords or straps may be provided, to pass around each of the wearer's ears, or to extend around the back of the head, one cord passing above and one below the wearer's ears.

Alternatively, a single cord may pass twice across the back of the wearer's head. A first side of the support structure may be located, in use, adjacent to one of the wearer's ears while a second side of the support structure is located, in use adjacent to the other of the wearer's ears. The first and second sides of the support structure may be located in the regions of the support structure which, in use, are closest to the wearer's ears.

The two ends of the single elasticated cord or strap may be retained by a pair of first attachment formations positioned at a first side of the support frame, eg at a side adjacent to one of the wearer's ears. The first attachment formations may be integrally formed with the support frame, or may be formed separately and subsequently attached, for example by gluing or welding or a mechanical fastening (eg a snap-fit fastening). Each of the pair of first attachment formations may retain one end of the cord or strap, and may be a clip or clamp, or may be an aperture or slot through which the cord or strap may be threaded, with a knot being tied in the cord or strap to prevent it sliding back through the slot or aperture.

At least one, eg one, or two attachment formations may be positioned on a second side of the support frame, eg at a side adjacent to the other of the wearer's ears, which attachment formation(s) retain a centre portion of the cord or strap. The cord or strap may therefore extend from one of the first attachment formations, through the second attachment formation(s), such that a loop of cord is formed, and back to the other of the first attachment formations.

This arrangement may enable the cord or strap to be tightened by the user pulling the loop formed at the second attachment formation(s), thereby providing an improved tightening arrangement, which may be beneficial for other types of face mask.

According to an example, there is provided a face mask comprising a pair of first attachment formations and a pair of second attachment formations, the pair of first attachment formations being positioned on first side of the face mask which, in use, is located near a first ear of the wearer and the pair of second attachment formations being positioned on a second side of the face mask which, in use, is located near a second ear of the wearer, wherein each of the first pair of attachment formations provides means for securing an end portion of the strap, and the second pair of attachment formations provides means for releasably fixing an intermediate portion of the strap.

A pair of second attachment formations may be positioned on the second side of the support frame, and the cord or strap may pass sequentially through the pair of second attachment formations, such that a loop of cord is formed between the pair of second attachment formations. The second attachment formations may permit easy passage of the cord in one direction on application of tension, but present significant resistance to movement of the cord in the opposite direction without additional action by the user (eg depression of a button or application of a significantly larger force). Thus, the wearer may easily adjust the length of the strap to suit the size of their head with one hand by pulling on the loop of cord formed between the pair of second attachment formations while wearing the mask. On application of tension the cord will slide through the pair of second attachment formations, thus shortening the length of cord around the back of the wearer's head and urging the face mask against their face. When the fit is correct, the user releases the cord, and the mask is retained in place by the cord or strap.

The pair of second attachment formations may each comprise a slot or aperture, substantially covered by a tab. An edge of the tab and/or a corresponding edge of the slot or aperture may comprise means to grip the cord, eg a roughened surface or a series of teeth. In use, the cord or strap is inserted through the slot or aperture, between an edge of the tab and an edge of the slot or aperture. A gripping formation, such as a series of teeth or a roughened surface along at least one edge of the tab, or along corresponding edges of the slot/aperture and tab, may prevent the cord or elastic from sliding back through the slot or aperture. The series of teeth may be angled or curved outwardly, that is, in the direction in which easy movement of the cord through the slot or aperture is desired. Thus, the teeth may be angled or curved away from the slot or aperture, to encourage the teeth to embed into and grip the cord or strap when it moves in the reverse direction.

Alternatively, the pair of second attachment formations may each comprise a slot or aperture and a resilient member, the resilient member substantially covering the slot and being resiliently biased against it. In use, the cord or strap is inserted through the slot against the bias of the resilient member, such that tension applied to the strap pulls the resilient member away from the slot or aperture sufficiently to permit passage of the cord through the slot or aperture. When tension on the cord is released, the bias of the resilient member urges the resilient member back against the slot or aperture, occluding it and trapping the cord between the resilient member and an edge of the slot or aperture. Thus, the cord may be easily pulled through the slot by the application of tension against the bias of the resilient member, but the resilient member prevents inadvertent movement of the cord through the slot in the opposite direction, even when tension is applied to the cord (eg when the cord is stretched about the wearer's head). A means of releasing the biased member may be provided to permit the cord to slide through the slot in the reverse direction when desired by the wearer, eg a release catch, or movement in the reverse direction may be actuated when a significant force is applied by the wearer. The resilient member may further comprise means to grip the cord, eg a roughened surface or a series of teeth, to aid in preventing slippage of the cord.

The first and/or second attachment formations may form part of the support frame. The first and/or second attachment formations may be formed integrally with the support frame. The second attachment formations may be integrally formed with the support member, in a single moulding step. Thus, a tab extending in a planar direction across an aperture may be integrally formed within the support frame in a single moulding step. The tab may project from the support frame. Manufacturing limitations may result in the tab being attached to the aperture in undesirable locations by flashing, where the distance between an edge of the tab and an adjacent edge of the aperture is small, for instance is less than 2mm, or less than 1mm, or less than 0.5mm. This may be resolved by the application of force to the tab, to break the flashing. Force may be applied to a surface of the tab, for example to a surface of the tab which projects from the support frame. Such force may be applied to the tab using the overmoulding tool during the overmoulding step which forms the sealing member, thus reducing the number of separate steps required in the manufacture of the mask.

There is thus provided a method of integrally forming a tab extending in a planar direction across an aperture, such that the distance between at least one edge of the tab and at least one adjacent edge of the aperture is less than 2mm, said method comprising:
a) using injection moulding to form a tab which extends in a planar direction across an aperture, such that the distance between at least one edge of the tab and at least one adjacent edge of the aperture is less than 2mm;
b) applying force to a surface of the tab, to release flashing formed during the injection moulding process between said at least one edge of the tab and said at least one adjacent edge of the aperture.

When used in the manufacture of a filter mask, the injection moulding process in step a) above may also form the support frame, and the application of force in step b) above may be carried out by designing the shape of the overmoulding tool used in the manufacture of the sealing member such that, when the mould is closed over the support member prior to overmoulding, pressure is applied to the tab to break the flashing. Thus, this process may be used to integrally form the second attachment formations of the filter mask described herein, without the need for additional manufacturing steps.

Alternatively, the first and/or second attachment formations may be formed separately to the support frame and subsequently connected to the support frame by chemical or mechanical means.

The mask may comprise an aperture in the filter, the aperture may provide a conduit through the filter, or may include or be adapted to receive a further device, such as an exhalation valve. The aperture may bypass the filter material. The aperture in the filter material may be mounted and/or sealed to an aperture in the support frame or to a device mounted to the support frame. Furthermore, the polymeric material that is injected into the mould to form the sealing member may be brought into engagement with the filter and the aperture in the support frame or to the device mounted to the support frame, during injection moulding of the sealing member, in a manner that seals the filter about the aperture or device.

The mask may comprise one or more vents or valves, for example an exhalation valve. An exhalation valve may enable exhaled air to escape from the filter mask without passing through the filter, therefore reducing the heat and humidity retained by the filter, during use. However, the inclusion of an exhalation valve may not be desirable where the filter mask is needed to protect others from pathogens exhaled by the wearer, eg in a healthcare environment. The vent or valve may be located in the shield, or in the rim of the support frame. The valve may be inserted into an aperture in the shield or rim, or may form part of the shield or rim. Where the valve is inserted into or forms part of the shield, it may also extend through the filter material, and the overmoulding step which forms the sealing member may be used to additionally provide a seal between the valve and the filter material and/or between an aperture in the support frame for receiving the valve and the filter material. Where the valve is inserted into or forms part of the rim, a portion of the rim may be shaped to accommodate the valve, eg in the chin region.

Where a valve is included, the injection moulding step for forming the sealing member may also form the valve member. The sealing member and the valve member may therefore be integrally formed. After moulding of the sealing member/exhalation valve, the valve may be inserted either through openings in the filter and shield, or through an opening in the rim . Tension between the valve and the surrounding material (shield, filter or rim) biases it closed. This permits exhaled air to flow from the interior of the mask to the exterior, but prevents the flow of air in the opposite direction.

The support frame, eg the shield, and/or filter may be formed with an aperture to which either a releasable cover or an exhalation valve may be fitted. The overmoulding step which forms the sealing member may be used to additionally provide a seal between the aperture in the support frame for receiving the valve or cover and the filter material. For example, the releasable cover may be secured over the aperture using a snap-fit or screw fit connection, the cover being removed and replaced with an exhalation valve where required. This enables a single mask to be produced which may be used either with or without an exhalation valve. The size of the aperture will be an appropriate size for the attachment of an exhalation valve, and may be 10-50mm in diameter, or 15-40 mm in diameter, or 15-30mm in diameter. Typically, the diameter of the aperture will be approximately 20mm.

The filter mask may further comprise a spigot, which may be used to connect the mask to a tube for introducing gases to, or removing gases from, the mask and/or the wearer. Thus, the filter mask may be used as an oxygen delivery mask or respirator, with the additional protection that inhaled air (other than the oxygen being delivered) must pass through the filter. In the same way as with the exhalation valve, the spigot may be releasably attached to an aperture in the shield and, optionally, the filter. The aperture may be sized to accommodate a spigot having a diameter of from 10-50mm in diameter, or 15-40 mm in diameter, or 15-30mm in diameter. Alternatively, the aperture may be sized to accommodate a spigot having a diameter of from 2-10mm in diameter, or 2-8 mm in diameter, or 4-8mm in diameter. The spigot may alternatively be integrally formed with the shield, or with another part of the support frame.

Alternatively, the filter mask may not comprise a spigot. Such filter masks would be unsuitable for use as an oxygen delivery mask or a respiratory mask. The filtration mask may additionally or alternatively comprise an access port. For example, the filtration mask may comprise an access port for an endoscope, a nasal cannula, or a high flow nasal cannula, such that an endoscopy may be carried out or cannula worn while still providing the patient and medical practitioner with the protection offered by a filtration mask. Medicaments or oxygen may be delivered in through a high flow nasal cannula, and it is a feature of this type of treatment that aerosolised particles may be generated and released to the environment. Depending on the nature of the patient's illness, such aerosolised particles may be contaminated and pose a risk to those in the vicinity. Wearing a filter mask which allows for access to the airway to deliver a high flow nasal cannula but which contains any generated aerosol thus provides a safer environment for clinicians and other personnel in the vicinity. The access port may comprise an aperture in the filter mask. The aperture may comprise a pair of apertures in the support frame, eg shield, and sheet of filtering material that are in registration with each other, such that a cannula can pass through both apertures. The overmoulding step which forms the sealing member may be used to additionally provide a seal between the an aperture in the support frame and the filter material. The apertures in the support frame, shield and/or filtering material may be provided with removable covers, such that they may be covered when not in use.

The face mask may alternatively be constructed such that the sheet of filtering material is replaceable. This means that it would not be necessary to replace the entire mask after use, but only to replace the filtering material while the mask frame, comprising the support structure and seal, could be reused multiple times This reduces both cost and waste.

A mask having a replaceable filter according to this example may be produced using the same principles as have been previously described, in a method involving a single overmoulding step which fixes the relative positions of the support frame and seal.

According to an example, there is provided a method for the manufacture of a filter mask, the filter mask comprising a support frame and a sealing member, the support frame having a housing for receiving a filter, wherein the method comprises:
a) providing the support frame in a mould; and
b) injecting a polymeric material into the mould to form a first sealing member for sealing against the face of a wearer, and a second sealing member for sealing against the filter received by the housing,
wherein the sealing member is brought into engagement with the support frame, during injection moulding of the sealing member, in a manner that fixes the sealing member to the support frame.

According to an example, there is provided a filter mask manufactured according to the above described method. There is also provided a filter mask, the filter mask comprising a support frame and a sealing member, the support frame further comprising a housing for receiving a filter, wherein the sealing member is fixed to the support frame.

The filter may comprise a filter substrate only, which may be releasably attachable to the support frame by any suitable means, eg by clips, or by an interference fit, or by a releasable adhesive, eg an adhesive strip covered by a releasable liner that is removed immediately prior to insertion of the sheet of filtering material. Alternatively, the filter may comprise a filter substrate retained within a cartridge or other support, which is connectable to the support frame. For example, the cartridge may be connected to the external face of the support frame by one or more snap-fit connectors, or clips, or by a friction fit, or there may be a port provided in the support frame for the accommodation of the cartridge. In this embodiment, and where the mask comprises a shield, the shield may form part of the cartridge or other support (ie the cartridge or other support may comprise a shield) such that, when the cartridge is attached to the external face of the mask, the shield is positioned over the external surface of the filter. The shield may alternatively be hingedly attached to the support frame, such that it can be opened to permit insertion of the filter cartridge adjacent to the external face of the support frame, and subsequently closed. The filter cartridge may be retained in place by one or more clips or other attachment members, or the hingedly attached shield may retain the filter cartridge in place.

The filter cartridge alternatively be connected to the internal face of the support frame by one or more snap-fit connectors, or clips. In this embodiment, the shield (where present) may be present as shown in the drawings and as previously described. For example, filter mask may comprise a shield which, in use, extends over at least part of an outwardly-facing surface of the filter, at least part of the periphery of the shield being separated from the support frame, and/or which may be integrally formed with the support frame.

The cartridge may take the form of a rim of plastic, for example injection moulded plastic, which matches the shape and size of the filter substrate and which is fixed about the circumference of the filter substrate. Thus, the cartridge does not restrict the flow of air through the filter substrate.

Embodiments of the invention are now illustrated, by way of example only, with reference to the following drawings:
Figure 1 is a perspective view of a support frame of a first example of a filtration mask;
Figure 2 is a side view of the support frame of Figure 2;
Figure 3 is a perspective view of a filter of the first example of a filter mask
Figure 4a is a perspective view of the filter located within the support frame of the first embodiment of a filter mask according to the invention during manufacture;
Figure 4b is the view of Figure 4a, without the filter;

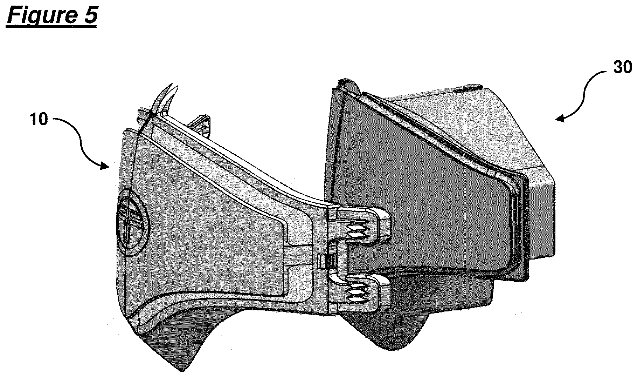

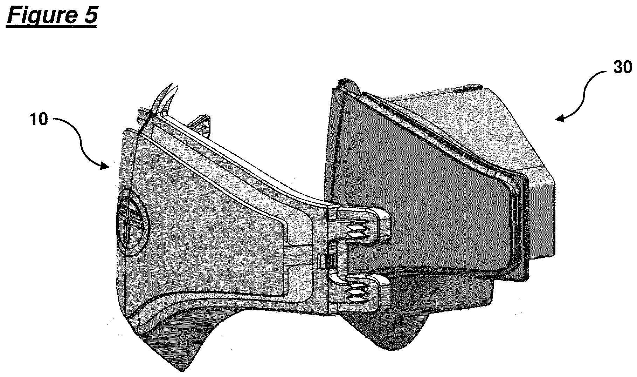

Figure 5 is a perspective view of the support frame and filter of Figure 4 and a core of the mould using during manufacture of the first example of a filter mask
Figure 6 is a side view of the first example of a filter mask
Figure 7 is a perspective view of the rear of the first example of a filter mask
Figure 8 is a schematic drawing showing the connection between the support frame and the filter material formed by overmoulding of the sealing member;
Figures 9a and 9b are perspective views of the front and rear of a second example of a filter mask; and
Figures 10a and 10b are perspective views of the front a rear of a third example of a filter mask.

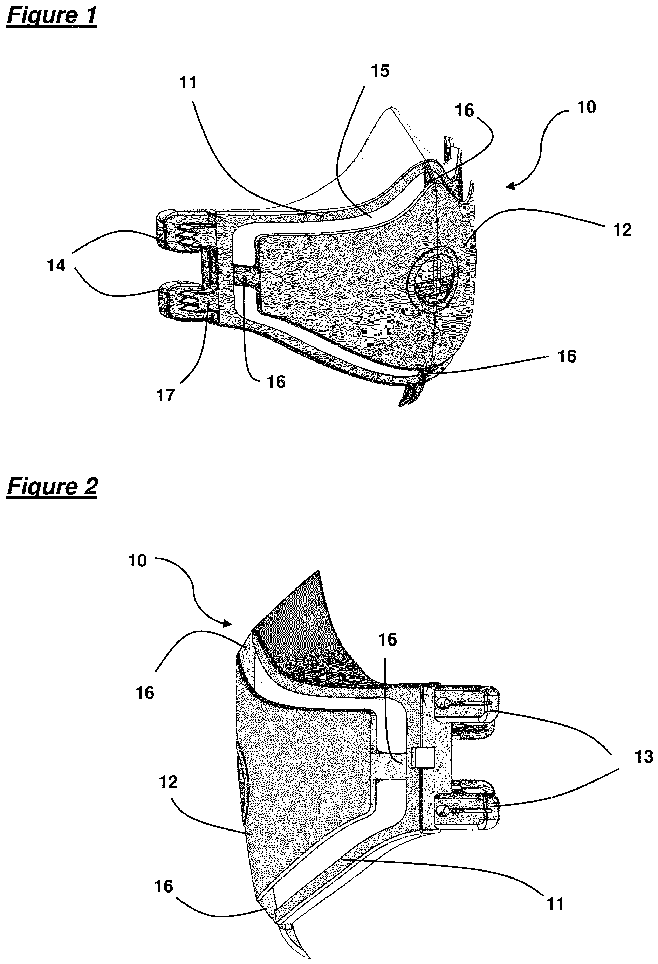

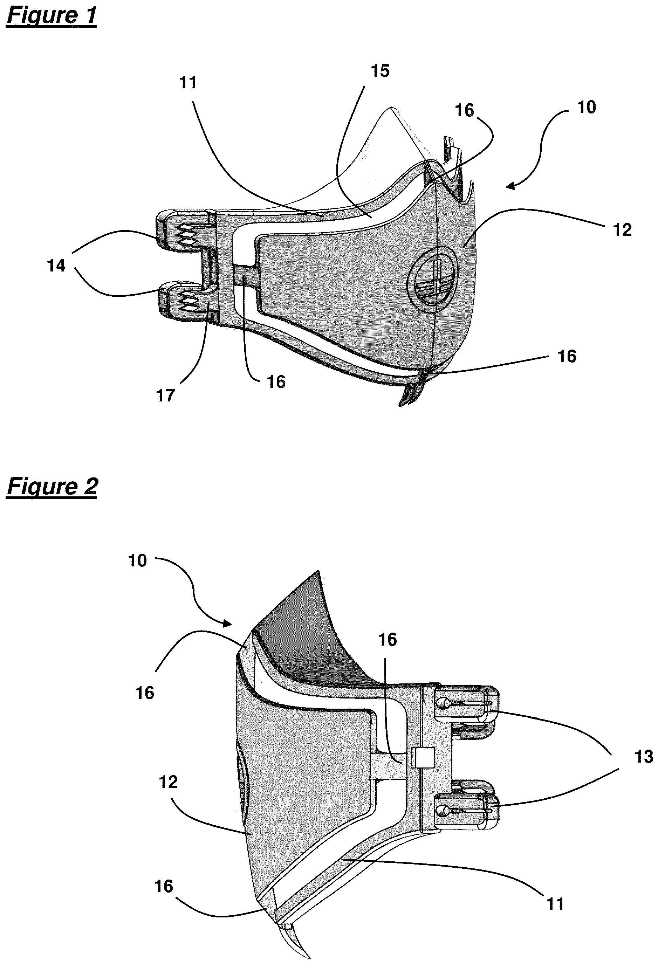

A support frame 10 for use in a filter mask is shown in Figures 1 and 2. The support frame 10 has a generally curved shape and comprises an integrally formed rim 11, a shield 12, a pair of first attachment formations 13 and a pair of second attachment formations 14. The shield 12 is substantially the same shape as the rim 11, but is smaller, and is offset from the rim 11 by approximately 1cm. The shield 12 is attached to the rim 11 by four connection members 16 equally spaced about the circumference of the rim, and attached to points close to the edge of the shield. A space 15 between the shield 12 and the rim 11 extends about the periphery of the shield, interrupted only by the connection members 16. The support structure 10 is an integrally formed component, with all features manufactured in a single injection moulding step.

A pair of first attachment formations 13 outwardly extend from a first side of the rim 11 which, in use, is located near a first ear of the wearer. The first attachment formations 13 take the form of a keyhole shaped slot through which a cord or strap (not shown) may be threaded, and secured with a knot or clamping device.

A pair of second attachment formations 14 extend outwardly from a second side of the rim which, in use, is located near the second ear of the wearer. The second attachment formations 14 comprise a slot (not visible) covered by a tab 17. The tab 17 has a series of teeth 18 which provide grip. The cord or strap (not shown) passes through the slot in the attachment formation 14 from the internal side of the support frame to the external side, between the tab 17 and an edge of the slot. The teeth 18 grip the cord and reduce or prevent movement of the cord in the reverse direction.

In use, the cord or strap is secured in the upper first attachment formation 13, passes through the upper second attachment formation 14 from the internal side to the external side, through the lower second attachment formation 14 from the external side to the internal side, and is finally secured in the lower first attachment formation 13. Thus, the strap passes behind the wearer's head twice, with a loop being formed between the pair of second attachment formations 14 on the external side of the support structure. When the wearer applies tension to this loop, eg by pulling the loop, the cord will be pulled through the apertures, past the teeth 18 on the tab 17. The release of this tension eg by the wearer releasing the loop of cord, will allow the teeth 18 to grip the cord, thus preventing the cord from sliding back through the slot unless a significant force is applied. In this way, one hand may be easily used to adjust the mask to fit the wearer's head.

Injection moulding is used to produce the support frame 10, the support frame 10 being an integrally formed unit produced from injection moulded polypropylene in a single step.

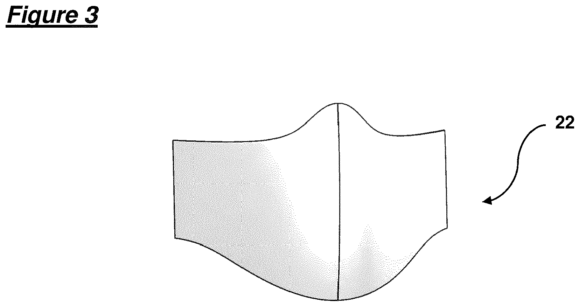

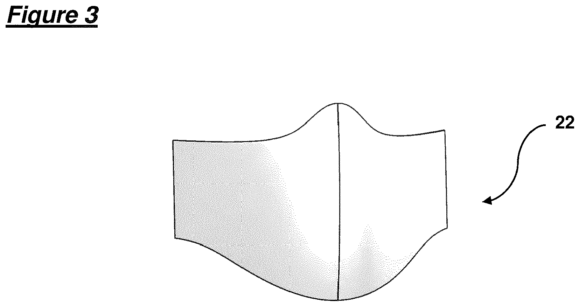

Where the manufacture of the filter mask is automated, the support frame 10 is ejected from the mould onto a vacuum cup on a robot arm. A pneumatic die pushes a sheet of filter material 22 from a roll of filtration material into the support frame 10, the roll of filter material comprising a continuous series of pre-stamped sheets of filter material, heat sealed about their peripheral edges. The sheet of filter material 22 is shown in Figure 3.

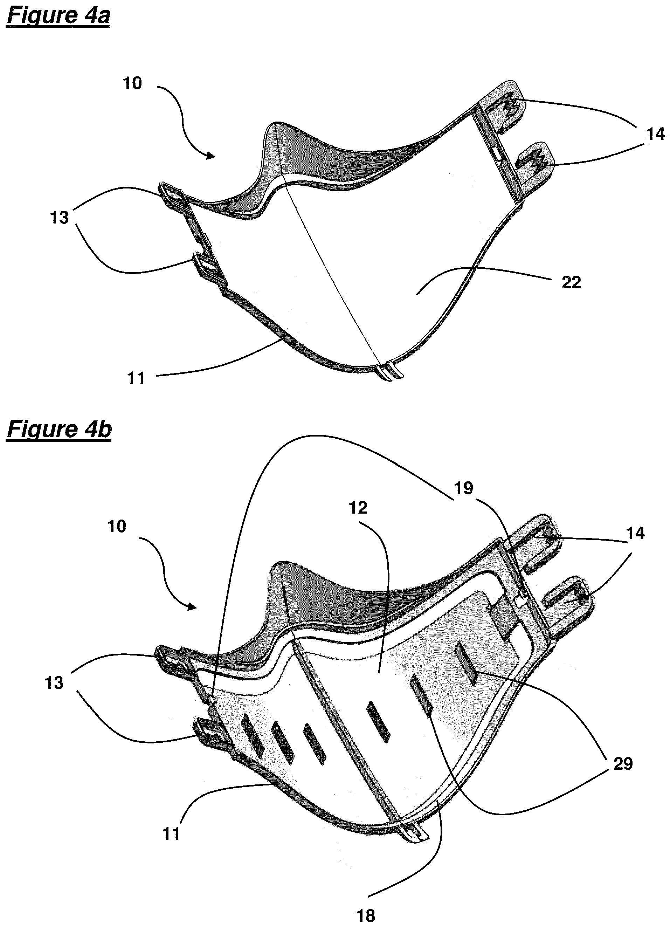

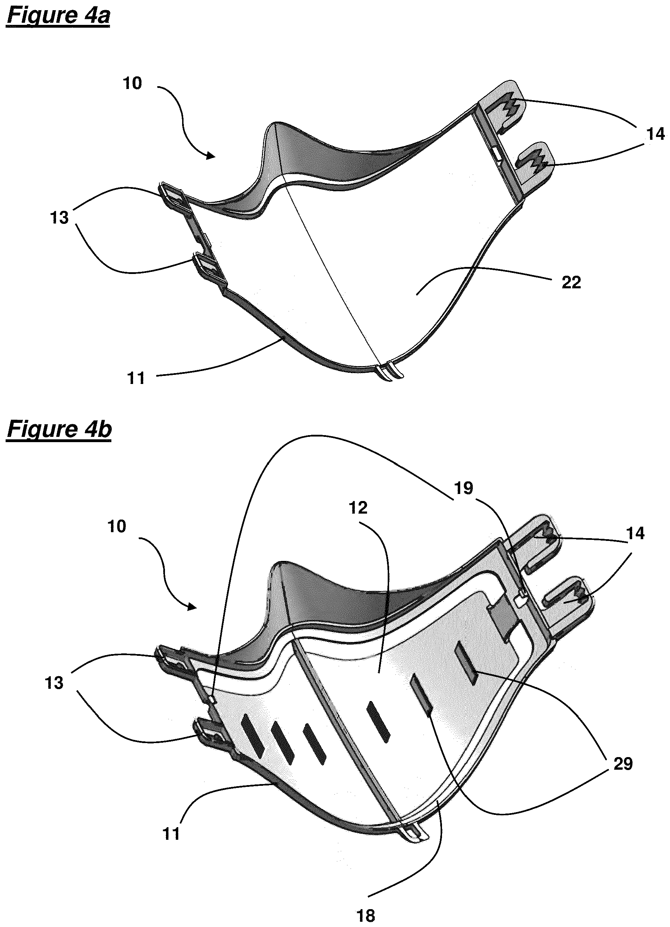

The sheet of filter material is positioned on the internal side of the support frame 10, that is, the side of the support frame 10 which will face the wearer in use. The sheet of filtering material is located on the inwardly extending shoulder, held in place about its periphery by retention tabs 19 at either side of the rim. This support structure/filter material assembly is shown in Figures 4a and 4b.

The support structure/filtration material assembly is transferred into a first half of the mould and mounted to a mould core 30. A second half of the mould is sealed against the first half of the mould, forming the mould cavity. In an injection moulding step, a sealing member 25 is overmoulded to the support structure/filtration material assembly, surrounding the periphery of the sheet of filter material 22 and adhering it to the support frame 10, and forming the sealing member 25, in a single overmoulding step. The filter mask 20 is removed from the mould, eg using a large 20mm pin to unpeel the filter mask 20 from the core 30, and transferred for manual attachment of an elasticated cord and subsequent packaging.

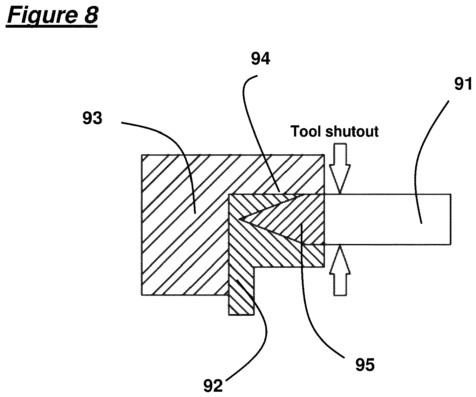

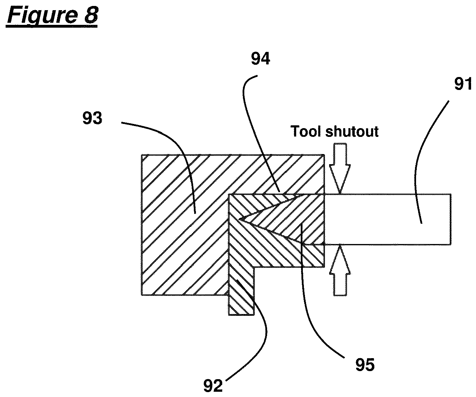

The connection between the filter, sealing member and support frame is shown schematically in Figure 8. The periphery of the filter 91 is placed against a shoulder 94 extending laterally from the rim of the support frame 93, either before or after the support frame is placed in the mould. A thermoplastic elastomer (TPE) 92 is injected into the mould to form the sealing member. The portion of TPE 92 shown in Figure 8 represents only a proximal edge of the sealing member. The TPE 92 is forced by the mould between the peripheral edge of the filter 91 and the rim and shoulder of the support frame 93, 94. The TPE 92 surrounds and impregnates the peripheral edge of the filter 91, the peripheral, impregnated region being denoted 95, adhering it to the rim and shoulder of the support frame 93, 94. Tool shutout, as shown in Figure 8, prevents ingress of TPE 92 into the filter 91 beyond its periphery 95, ensuring that the filtering action of the filter 91 is not inhibited by TPE. As well as aiding adhesion of the support frame 93, 94 to the filter, impregnation of the peripheral edge 95 of the filter 91 with TPE may contribute to the seals at the edge of the filter, formed by heat sealing, preventing moisture or other contaminants from seeping into the structure of the filter.

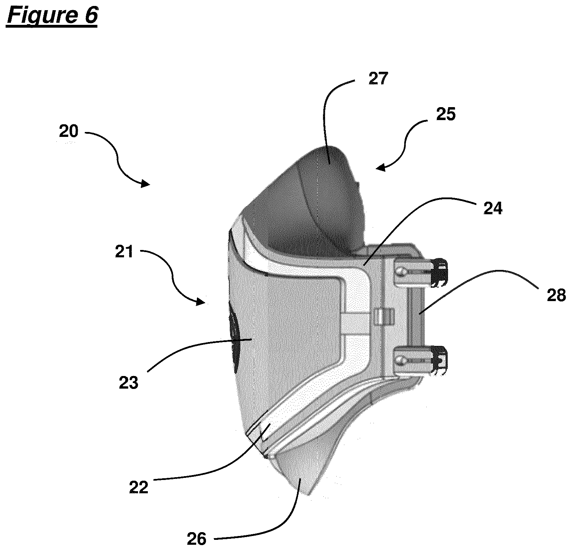

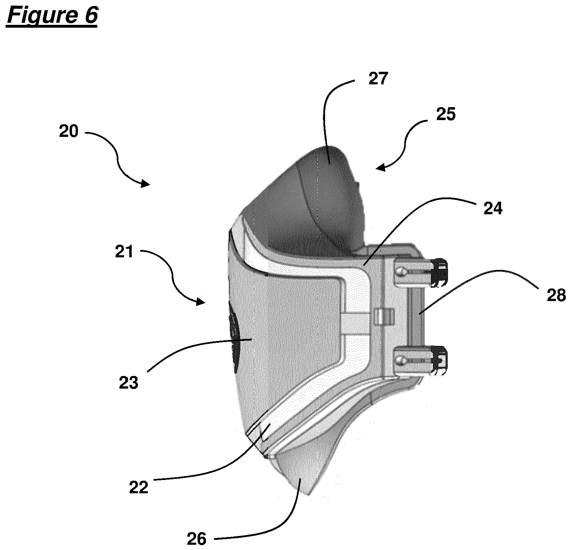

A side view of a filtration mask 20 is shown in Figure 6.

The support structure 21 is the same as that described in relation to Figures 1 and 2. A sheet of filtering material 22 is positioned behind the support structure 21, and is visible in the gap between the shield 23 and the rim 24, about the periphery of the shield 23. This permits air to easily flow past the shield 23 and through the filtering material 22, while still benefitting from the protection of the shield across the filtering material.

The sealing member 25 is overmoulded onto the support frame 21 and filter material 22, fixing their relative positions. The sealing member 25 comprises a chin engaging portion 26, a nose engaging portion 27 and cheek engaging portions 28. The cheek engaging portions 28 extend laterally across the cheeks, forming a seal against the softer skin on the cheek and improving the overall seal of the mask.

The sealing member 25 is formed of an elastomeric material. It provides a compressible region between the support structure 21 and the wearer's face, enabling the elastomeric material to conform to the contours of the wearer's face and provide a good seal against it.

Figures 4a and 4b show the support structure/filter member assembly, with and without the filter material present respectively. The support structure 10 comprises a rim 11. The rim 11 has an inwardly extending shoulder 18 onto which a pre-cut sheet of filter material 22 is placed. Extending from a first side of the rim are first attachment formations 13, and second attachment formations 14 extend from the second side of the rim. The support frame 10 also comprises a shield 12. A series of vertical ribs 29 across the back of the shield 12 ensure that the spacing between the shield 12 and the filter material 22 is maintained.

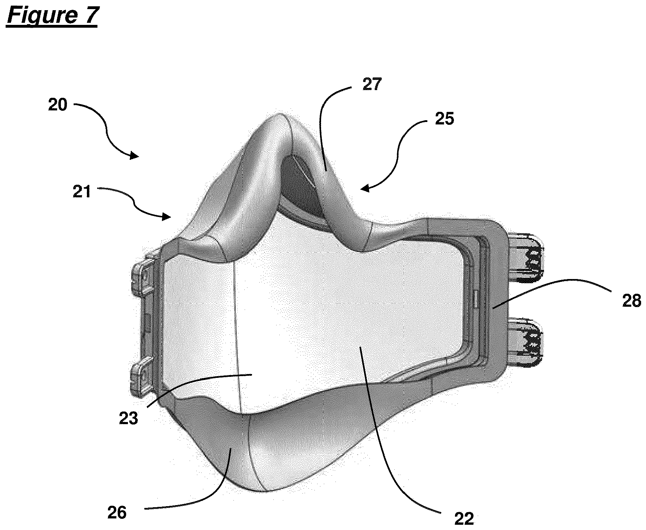

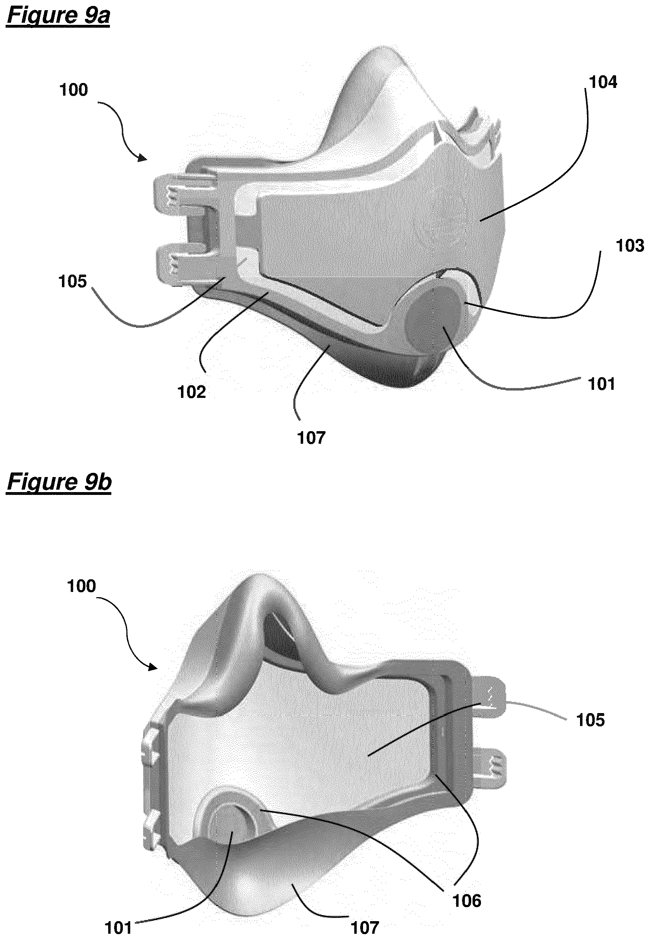

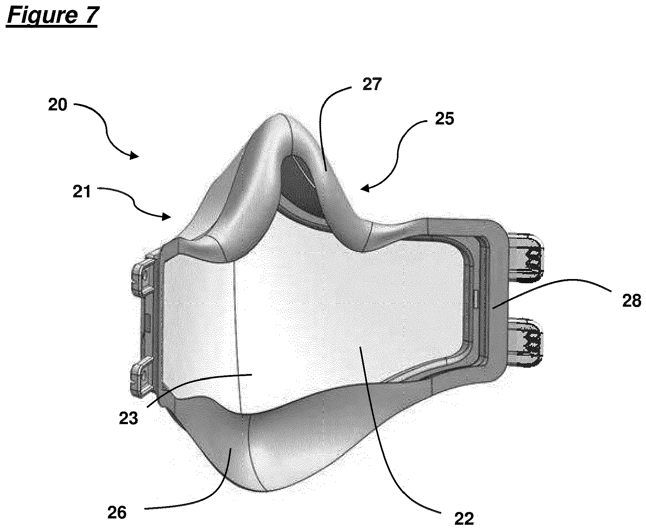

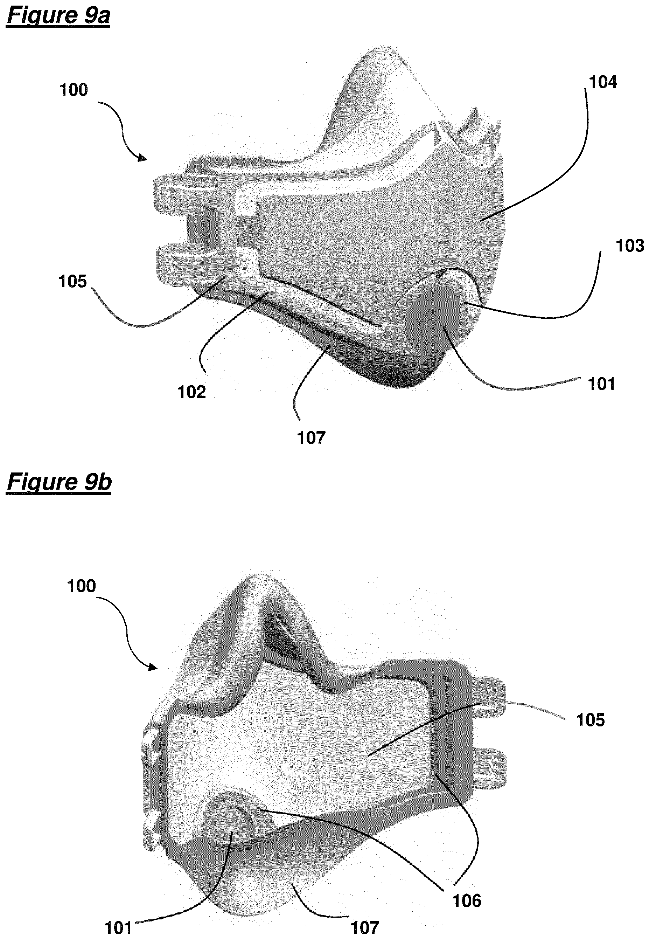

A second example of a filter mask can be seen in Figures 9a and 9b, and is generally designated 100. The structure of the mask is substantially the same as that described in relation to the first example depicted in Figures 6 and 7, except that the mask 100 further comprises a mushroom valve 101 (shown in the closed position). In the chin region, the rim 102 of the support frame comprises an upwardly projecting portion 103, and the shape of the shield 104 and filter material 105 corresponds to the shape of the inner periphery of the rim 102 such that they accommodate the upwardly projecting portion 103. The filter material 105 is sealed along the periphery of the rim 102 of the support frame, including the upwardly projecting portion 103, by a surrounding rim of TPE 106. The upwardly projection portion comprises an aperture, with the mushroom valve 101 being formed in the aperture as part of the overmoulding step which also forms the sealing member 107. The mushroom valve 101 permits exhaled air to escape without passing through the filter material 105. The mushroom valve 101 could alternatively be accommodated within the shield 104.

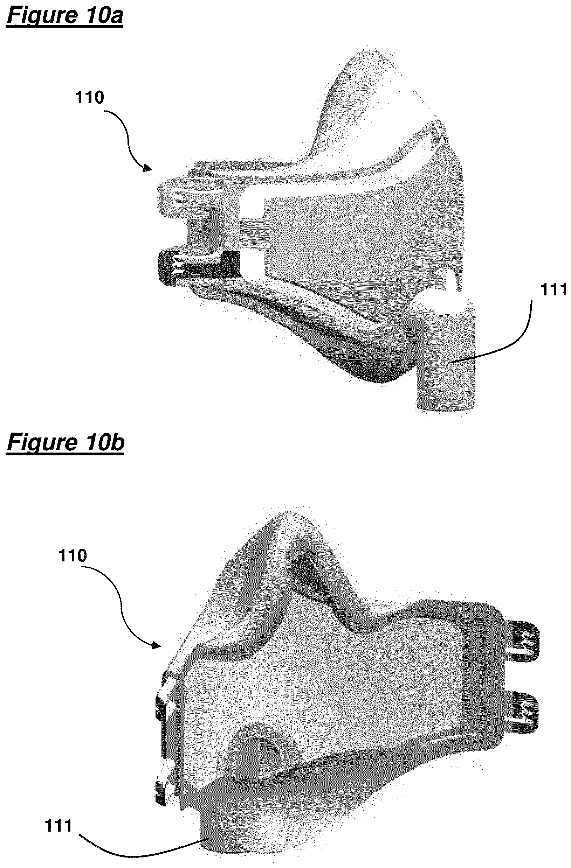

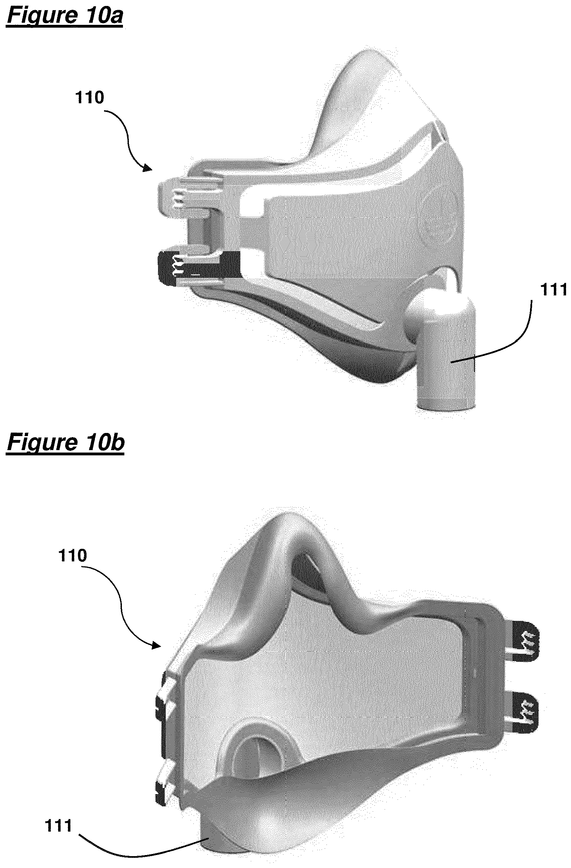

The third example of a filter mask shown in Figures 10a and 10b, which is generally denoted 110, has substantially the same structure is described in the second embodiment in relation to Figures 9a and 9b, but shows an elbow connection 111 in place of the mushroom valve. The elbow connection 111 permits connection of the mask 110 to other medical equipment, such as a nebuliser.

## Claims

1. A method of manufacturing a filter mask, the filter mask comprising a support frame (10), a filter (22) and a sealing member (25), wherein the method comprises:
a) locating the filter and the support frame in a mould; and
b) injecting a polymeric material into the mould to form the sealing member,
**characterised in that** the sealing member is brought into engagement with the filter and
the support frame, during injection moulding of the sealing member, in a manner that fixes the sealing member to the support frame and the filter, and in a manner that fixes the relative positions of the filter and the support frame.

2. A method of manufacturing a filter mask as claimed in Claim 1, wherein the support frame is made in a preceding step of the method by injection moulding, and the method further comprises a step of transferring the support frame from a first mould in which the support frame is injection moulded to a second mould in which the sealing member is injection moulded.

3. A method of manufacturing a filter mask as claimed in Claim 1 or Claim 2, wherein when the filter is located into the mould with the support frame, the support frame extends about the periphery of the filter.

4. A method of manufacturing a filter mask as claimed in any preceding claim, wherein the support frame comprises a rim that defines an aperture through which inhaled and/or exhaled gases flow, in use.

5. A method of manufacturing a filter mask as claimed in Claim 4, wherein the support frame comprises a shoulder extending from an inner edge of the rim, providing a surface on which the filter can be located, facilitating retention of the filter in the mould prior to injection moulding of the sealing member.

6. A method of manufacturing a filter mask as claimed in Claim 4 or Claim 5, wherein the filter is located on the support frame before the support frame and filter are together located in the mould.

7. A method of manufacturing a filter mask as claimed in any preceding claim, wherein the support frame comprises one or more ribs, which extend across a surface of the filter, providing support to the filter and rigidity to the mask.

8. A method of manufacturing a filter mask as claimed in any preceding claim, wherein the filter that is located in the mould in the method of manufacture comprises a filter substrate only.

9. A method of manufacturing a filter mask as claimed in any preceding claim, wherein the support frame and the filter are not fixed or bonded together prior to injection moulding of the sealing member.

10. A method of manufacturing a filter mask as claimed in any preceding claim, wherein, during injection moulding of the sealing member, a portion of the sealing member adheres aligned edges of the filter and the support frame.

11. A filter mask manufactured according to the method as claimed in any preceding claim.

12. A filter mask comprising a support frame (10), a filter (22) and a sealing member (25), **characterised in that** the sealing member is fixed to the support frame and the filter, and fixes the relative positions of the filter and the support frame.

13. A filter mask as claimed in Claim 12, wherein an external face of the support frame comprises a shield, which extends over at least part of a surface of the filter.

14. A filter mask as claimed in Claim 13, wherein the shield is separated from the filter, such that air may flow between the shield and the filter before the air is drawn through the filter by the wearer during inhalation.

15. A filter mask as claimed in Claim 13 or Claim 14, wherein one or more inlet apertures, through which air may be drawn by a wearer inhaling, is defined between an edge of the shield and the support frame.

## Patentansprüche

1. Herstellungsverfahren von einer Filtermaske, wobei die Filtermaske einen Stützrahmen (10), einen Filter (22) und ein Dichtungselement (25) umfasst, wobei das Verfahren Folgendes umfasst:
a) Platzieren des Filters und des Stützrahmens in einer Form, und
b) Einspritzen eines Polymermaterials in die Form, um das Dichtungselement zu bilden,
**dadurch gekennzeichnet, dass**
das Dichtungselement auf eine Weise, die das Dichtungselement an dem Stützrahmen und dem Filter fixiert, und auf eine Weise, welche die relativen Positionen des Filters und des Stützrahmens fixiert, während des Spritzgießens des Dichtungselements mit dem Filter und dem Stützrahmen in Eingriff gebracht wird.

2. Herstellungsverfahren von einer Filtermaske nach Anspruch 1, wobei der Stützrahmen in einem vorhergehenden Schritt des Verfahrens durch Spritzgießen gefertigt wird und wobei das Verfahren ferner einen Schritt des Übertragens des Stützrahmens von einer ersten Form, in welcher der Stützrahmen spritzgegossen wird, in eine zweite Form umfasst, in der das Dichtungselement spritzgegossen wird.

3. Herstellungsverfahren von einer Filtermaske nach Anspruch 1 oder Anspruch 2, wobei sich der Stützrahmen um den Umfang des Filters erstreckt, wenn der Filter mit dem Stützrahmen in der Form platziert wird.

4. Herstellungsverfahren von einer Filtermaske nach einem der vorhergehenden Ansprüche, wobei der Stützrahmen einen Rand umfasst, der eine Öffnung bildet, durch die bei Gebrauch eingeatmete und/oder ausgeatmete Gase strömen.

5. Herstellungsverfahren von einer Filtermaske nach Anspruch 4, wobei der Stützrahmen eine Schulter umfasst, die sich von einer Innenkante des Randes erstreckt und eine Fläche bereitstellt, auf welcher der Filter platziert werden kann, wodurch ein Festhalten des Filters in der Form vor dem Spritzgießen des Dichtungselements erleichtert wird.

6. Herstellungsverfahren von einer Filtermaske nach Anspruch 4 oder Anspruch 5, wobei der Filter auf dem Stützrahmen platziert wird, bevor der Stützrahmen und der Filter gemeinsam in der Form platziert werden.

7. Herstellungsverfahren von einer Filtermaske nach einem der vorhergehenden Ansprüche, wobei der Stützrahmen eine oder mehrere Rippen umfasst, die sich über eine Fläche des Filters erstrecken und dem Filter Stützung und der Maske Stabilität bereitstellen.

8. Herstellungsverfahren von einer Filtermaske nach einem der vorhergehenden Ansprüche, wobei der Filter, der bei dem Herstellungsverfahren in der Form platziert wird, nur ein Filtersubstrat umfasst.

9. Herstellungsverfahren von einer Filtermaske nach einem der vorhergehenden Ansprüche, wobei der Stützrahmen und der Filter vor dem Spritzgießen des Dichtungselements nicht aneinander fixiert oder miteinander verbunden sind.

10. Herstellungsverfahren von einer Filtermaske nach einem der vorhergehenden Ansprüche, wobei während des Spritzgießens des Dichtungselements ein Abschnitt des Dichtungselements an ausgerichteten Kanten des Filters und des Stützrahmens haftet.

11. Filtermaske, die nach dem Verfahren gemäß einem der vorhergehenden Ansprüche hergestellt wurde.

12. Filtermaske, umfassend einen Stützrahmen (10), einen Filter (22) und ein Dichtungselement (25), **dadurch gekennzeichnet, dass**
das Dichtungselement an dem Stützrahmen und an dem Filter fixiert ist und die relativen Positionen des Filters und des Stützrahmens fixiert.

13. Filtermaske nach Anspruch 12, wobei eine Außenseite des Stützrahmens einen Schild umfasst, der sich über mindestens einen Teil einer Fläche des Filters erstreckt.

14. Filtermaske nach Anspruch 13, wobei der Schild von dem Filter getrennt ist, sodass Luft zwischen dem Schild und dem Filter strömen kann, bevor die Luft während des Einatmens von dem Träger durch den Filter eingesaugt wird.

15. Filtermaske nach Anspruch 13 oder Anspruch 14, wobei zwischen einer Kante des Schildes und dem Stützrahmen eine oder mehrere Einlassöffnungen definiert sind, durch die der Träger beim Einatmen Luft einsaugen kann.

## Revendications

1. Méthode de fabrication d'un masque à filtrer, le masque à filtrer comprenant un cadre support (10), un filtre (22) et un élément d'étanchéité (25), ladite méthode comprenant :
a) la mise en place du filtre et du cadre support dans un moule ; et
b) l'injection d'un matériau polymère dans le moule pour former l'élément d'étanchéité,
**caractérisée en ce que**
l'élément d'étanchéité est amené en prise avec le filtre et le cadre support, pendant le moulage par injection de l'élément d'étanchéité, d'une manière qui fixe l'élément d'étanchéité au cadre support et au filtre, et d'une manière qui fixe les positions relatives du filtre et du cadre support.

2. Méthode de fabrication d'un masque à filtrer selon la revendication 1, ledit cadre support étant fabriqué dans une étape précédente de la méthode par moulage par injection, et ladite méthode comprenant en outre une étape de transfert du cadre support d'un premier moule dans lequel le cadre support est moulé par injection vers un second moule dans lequel l'élément d'étanchéité est moulé par injection.

3. Méthode de fabrication d'un masque à filtrer selon la revendication 1 ou la revendication 2, lorsque le filtre est placé dans le moule avec le cadre support, le cadre support s'étendant autour de la périphérie du filtre.

4. Méthode de fabrication d'un masque à filtrer selon l'une quelconque des revendications précédentes, ledit cadre support comprenant un rebord qui définit une ouverture à travers laquelle s'écoulent les gaz inhalés et/ou expirés, lors de l'utilisation.

5. Méthode de fabrication d'un masque à filtrer selon la revendication 4, ledit cadre support comprenant un épaulement s'étendant à partir d'un bord intérieur du rebord, fournissant une surface sur laquelle le filtre peut être placé, facilitant la rétention du filtre dans le moule avant le moulage par injection de l'élément d'étanchéité.

6. Méthode de fabrication d'un masque à filtrer selon la revendication 4 ou la revendication 5, ledit filtre étant placé sur le cadre support avant que le cadre support et le filtre ne soient placés ensemble dans le moule.

7. Méthode de fabrication d'un masque à filtrer selon l'une quelconque des revendications précédentes, ledit cadre support comprenant une ou plusieurs nervures, qui s'étendent sur une surface du filtre, assurant un support au filtre et une rigidité au masque.

8. Méthode de fabrication d'un masque à filtrer selon l'une quelconque des revendications précédentes, ledit filtre qui est placé dans le moule dans la méthode de fabrication comprenant uniquement un substrat de filtre.

9. Méthode de fabrication d'un masque à filtrer selon l'une quelconque des revendications précédentes, ledit cadre support et ledit filtre n'étant pas fixés ou liés ensemble avant le moulage par injection de l'élément d'étanchéité.

10. Méthode de fabrication d'un masque à filtrer selon l'une quelconque des revendications précédentes, pendant le moulage par injection de l'élément d'étanchéité, une partie de l'élément d'étanchéité adhérant aux bords alignés du filtre et du cadre support.

11. Masque à filtrer fabriqué conformément à la méthode selon l'une quelconque des revendications précédentes.

12. Masque à filtrer comprenant un cadre support (10), un filtre (22) et un élément d'étanchéité (25), **caractérisé en ce que**
l'élément d'étanchéité est fixé au cadre support et au filtre, et fixe les positions relatives du filtre et du cadre support.

13. Masque à filtre selon la revendication 12, une face externe du cadre support comprenant une protection, qui s'étend sur au moins une partie d'une surface du filtre.

14. Masque à filtrer selon la revendication 13, ladite protection étant séparée du filtre, de sorte que l'air puisse circuler entre la protection et le filtre avant que l'air ne soit aspiré à travers le filtre par le porteur pendant l'inhalation.

15. Masque à filtrer selon la revendication 13 ou la revendication 14, une ou plusieurs ouvertures d'entrée, à travers lesquelles l'air peut être aspiré par un porteur en train d'inspirer, étant définies entre un bord de la protection et le cadre support.
